Europäisches Patentamt

**European Patent Office**

**Office européen des brevets**

(11) Numéro de publication: **0 171 337**

**B1**

(12) **FASCICULE DE BREVET EUROPÉEN**

(45) Date de publication du fascicule du brevet:
**02.08.89**

(51) Int. Cl.⁴: **A 61 M 5/14**

(21) Numéro de dépôt: **85420128.2**

(22) Date de dépôt: **12.07.85**

(54) **Appareil pour administrer un liquide au moyen d'un injecteur.**

(30) Priorité: **07.08.84 IT 5371084 U**

(43) Date de publication de la demande:
**12.02.86 Bulletin 86/7**

(45) Mention de la délivrance du brevet:
**02.08.89 Bulletin 89/31**

(84) Etats contractants désignés:
**BE CH DE FR GB IT LI NL SE**

(56) Documents cité:
**DE-A-2 724 538**
**DE-A-3 307 810**
**GB-A-2 115 495**
**US-A-4 132 231**

(73) Titulaire: **HOSPAL AG, Missionsstrasse 60/62, CH-4012 Basel (CH)**

(72) Inventeur: **Aldrovandi, Mauro, Via Statale Nord 7/B, I-41037 Mirandola (Modena) (IT)**

(74) Mandataire: **Gauckler, Jacques, Service Brevets HOSPAL HOSPAL C.O.T. B.P. 21, F-69881 Meyzieu Cedex (FR)**

LIBER, STOCKHOLM 1989

## Description

La présente invention concerne un appareil pour administrer un liquide au moyen d'un injecteur. En particulier, la présente invention est relative à un appareil capable d'administrer au moyen d'un injecteur, une quantité relativement faible (20 - 30 cm³) d'un liquide dans un temps relativement long (3 - 4 heures).

Les appareils capables d'administrer un liquide selon les modalités exposées ci-avant sont avantageusement utilisés dans le domaine médical, par exemple dans les traitements de dialyse du sang pour administrer graduellement au patient une solution héparinée, durant toute la durée du traitement qui est en général de trois à quatre heures. Le liquide à administrer est préalablement introduit dans la chambre d'un injecteur muni d'un piston. L'appareil possède des moyens d'ancrage du corps de l'injecteur et des moyens de traction du piston dans une direction axiale intérieure à ladite chambre.

Les inconvénients les plus sérieux lors de l'utilisation de tels appareils sont dus à une obstruction indésirée du conduit à l'aval de l'appareil lui-même. En fait une obstruction non remarquée peut être préjudiciable au patient, puisque l'infusion s'interrompt et peut aussi entraîner des dommages avec rupture possible de la ligne d'administration du liquide à cause de la forte augmentation de pression du liquide poussé par le piston précité.

Dans le but d'éviter de tels inconvénients, les moyens actuels de traction du piston présentent, en position perpendiculaire au sens d'avancement du piston, un plateau d'accouplement frontal comprenant deux engrenages à dents triangulaires poussés l'un contre l'autre par des ressorts. De cette manière, une augmentation éventuelle de la pression du liquide due à une obstruction du conduit d'infusion provoque, en conséquence, le désaccouplement des engrenages et le déclenchement par l'un d'eux d'un micro-interrupteur relié à un circuit d'alarme convenable.

On observe cependant que le relèvement de la surpression ainsi obtenue est insuffisamment fiable et non constant dans le temps puisqu'il dépend de l'usure des dents des engrenages et du degré d'accouplement des engrenages eux-mêmes, qui varie selon la position relative de ces derniers.

En outre, de tels appareils doivent être munis d'un micro-interrupteur supplémentaire pour signaler la fin de la course du piston.

Un exemple de dispositif d'injection d'un liquide selon l'art antérieur est décrit dans la demande GB-2 115 495. Le titulaire de cette demande a essayé de combiner les systèmes d'alarme correspondant à la détection de fin de course du piston dans la chambre du liquide à injecter, et à la détection d'une obstruction dans le conduit d'infusion du liquide. Les deux systèmes de sécurité sont combinés car le déclenchement du dispositif d'alarme et l'arrêt du moteur sont provoqués par l'ouverture d'un micro-interrupteur commun.

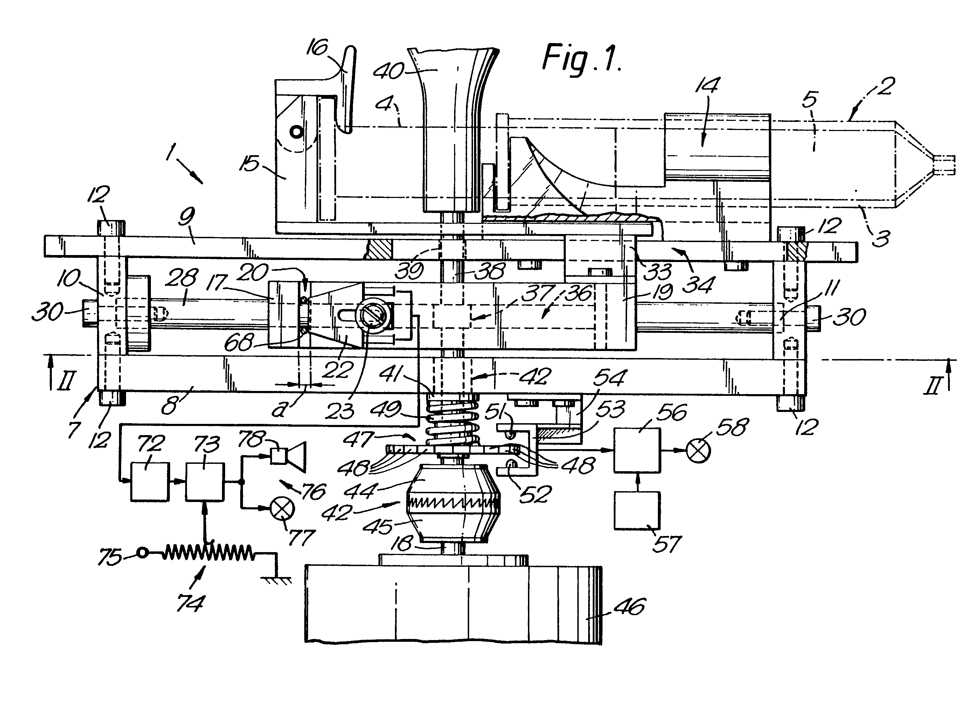

Cependant, si l'action finale d'ouverture du micro-interrupteur est commune aux deux causes de déclenchement du système de sécurité, les voies d'action sur cet interrupteur sont complètement indépendantes. En effet, ainsi que cela apparait sur la figure 1, la fin de course du piston est détectée grâce à l'action d'une tige 25 sur le micro-interrupteur cette tige 25 prolonge le support 16 qui entraine la traction du piston grâce à l'action d'un moteur 19.

Par contre, une obstruction du conduit d'infusion est détectée par la surpression s'exerçant sur un ressort 11. Ce ressort est lié à la branche 10 du support sur laquelle est fixée la tête d'injection de la seringue.

L'un des systèmes de détection est donc dépendant de la pression s'exerçant sur le support de la tête d'injection de la seringue, alors que l'autre système est lié au déplacement de la branche du support entrainant le déplacement du piston.

Ainsi, l'intégration dans un système unique de la détection de l'une ou l'autre des causes de déclenchement du dispositif de sécurité, n'est pas, ainsi que cela aurait été souhaitable, réalisée dans ce dispositif de l'art antérieur.

Un but de la présente invention est de proposer un appareil pour administrer un liquide au moyen d'un injecteur, qui surmonte les inconvénients des appareils de types connus et énumérés ci-avant.

Ce but est atteint par la présente invention qui est relative à un appareil pour administrer un liquide contenu dans la chambre d'un injecteur muni d'un piston coulissant dans ladite chambre, l'appareil présentant des moyens d'ancrage du corps de l'injecteur et des moyens de traction du piston en direction axiale à l'intérieur de ladite chambre, lesdits moyens de traction comprenant un premier élément accouplé à un organe de transmission de mouvement et un second élément capable de coopérer avec ledit piston pour le tirer axialement, caractérisé en ce que lesdits moyens de traction comprennent:

- des moyens de liaison qui établissent entre lesdits premier et second éléments un accouplement élastique avec une course (a) de longeur prédéterminée dans la direction d'avancement du piston à l'intérieur de ladite chambre,

- des moyens permettant de mesurer à tout instant la distance prise suivant la direction d'avancement du piston entre lesdits premier et second éléments, lesdits moyens comprenant un élément transducteur capable d'émettre un signal électrique dépendant de la distance entre lesdits premier et second éléments,

ledit appareil comprenant en outre des moyens de comparaison dudit signal émis par ledit transducteur avec un signal de référence dont la valeur est fonction d'une distance prédéterminée entre lesdits premier et second éléments pour émettre un signal de sortie, ainsi que des moyens de signalisation reliés à la sortie desdits moyens de comparaison et susceptibles d'être excités ou désexcités par ledit signal de sortie pour indiquer l'obtention de ladite distance prédéterminée.

Pour une meilleure compréhension de la présente invention, on en décrit maintenant une forme préférée de réalisation, à simple titre d'exemple non limitatif et en se référant aux dessins ci-joints dans lesquels:

* la figure 1 est une vue de dessus et en partie schématique d'un appareil réalisé selon les enseignements de la présente invention,
* La figure 2 est une vue en élévation et en coupe selon II-II de la figure 1.

En se référant plus particulièrement à la figure 1, la référence (1) désigne l'ensemble de l'appareil pour administrer un liquide au moyen d'un injecteur (2). Ce dernier présente en particulier un corps (3) essentiellement cylindrique à l'intérieur duquel coulisse un piston (4) qui délimite avec le corps (3) une chambre (5) à l'intérieur de laquelle est contenu le liquide à administrer. L'appareil (1) possède un chassis support (7) constitué essentiellement de quatre plaques rectangulaires, respectivement (8), (9), (10) et (11), montées face à face deux à deux et reliées entre elles au moyen de vis (12).

Sur la plaque (9) on fixe extérieurement un élément de type connu (14) capable de constituer un ancrage stable au corps (3) de l'injecteur (2) à la plaque support (9) elle-même. Le piston (4), quant à lui, est apte à être accroché à un élément (15) en forme de L à l'extrémité libre duquel peut pivoter un crochet (16) susceptible de coopérer avec l'extrémité du piston (4) pour verrouiller celui-ci contre ladite extrémité libre de l'élément (15). De cette façon le piston (4) peut être entraîné dans une direction axiale et dans les deux sens grâce à l'élément (15).

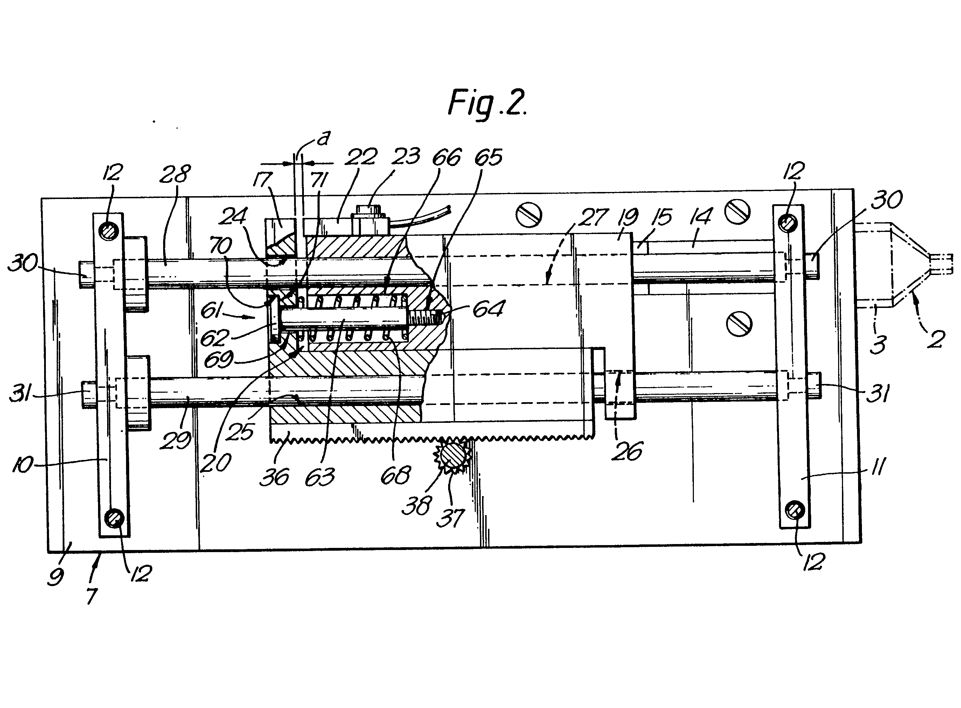

Selon la présente invention, l'appareil (1) comprend un premier élément (17) relié à un organe de transmission de mouvement (18), un second élément (19) solidaire de l'élément de traction (15) en forme de L du piston (4) et des moyens de liaison (20) (voir aussi la figure 2) qui établissent entre les premier et second éléments (17, 19) un accouplement élastique avec la possibilité d'un déplacement relatif maximum (course) (a) dans la direction d'avancement du piston (4) à l'intérieur de la chambre (5) de l'injecteur (2). L'appareil (1) comprend en outre des moyens de mesure (22) capables de relever la distance axiale entre les éléments (17) et (19). En particulier de tels moyens de mesure peuvent être constitués essentiellement par un dispositif optique à réflexion fixé sur l'élément (19) au moyen d'une vis (23) et faisant face à l'élément (17).

En se référant plus particulièrement à la figure 2, les éléments (17) et (19) ont essentiellement la forme d'un L. En outre, chacun d'eux présente deux trous parallèles, respectivement (24, 25) et (26, 27), disposés parallèlement et perpendiculairement aux bras composant chacun desdits éléments en L. Les éléments (17) et (19) en forme de L sont essentiellement complémentaires et sont montés de manière telle que les trous (24) et (27) puis (25) et (26) sont disposés respectivement le long d'un même axe et sont engagés dans des tiges guide cylindriques respectivement (28) et (29), fixées parallèlement sur les plaques (10) et (11) du chassis (7) au moyen de vis respectivement (30) et (31).

Comme on le voit bien figure 1, le second élément (19) est rendu solidaire de l'élément (15) qui tire le piston (4), au moyen d'un etrier (33) qui coulisse à l'intérieur d'une rainure (34) creusée longitudinalement dans la plaque (9). En se référant aux figures 1 et 2, on fixe au premier élément (17) des moyens de traction tels qu' une crémaillère (36) sur la partie extérieure du bras percé du trou (25). Cette crémaillère s'engrène sur un pignon correspondant (37) calé sur un arbre (38). Ce dernier sort perpendiculairement à la plaque (9) à travers un orifice (39) percé dans celle-ci pour recevoir un pommeau (40) représenté partiellement figure 1.

L'arbre (38) est supporté par un palier (41) logé dans un trou correspondant ménagé dans la plaque (8) et il peut s'accoupler en rotation de manière amovible avec l'organe de transmission de mouvement (18) au moyen d'un plateau d'accouplement (42) muni de dents de scie dissymétriques comme représenté figure 1. Ce dernier comprend en particulier une première pièce (44) solidaire de l'arbre (38) et une seconde pièce (45) qui vient s'accoupler à l'organe (18) de transmission de mouvement, essentiellement constitué par l'arbre de sortie d'un moto-réducteur (46). L'arbre (38) porte en outre, calé dans la partie comprise entre la partie (44) et le palier (41) un disque (47) présentant à la périphérie une pluralité d'appendices radiaux équidistants (48). Entre le disque (47) et le palier (41), on place et on maintient en compression un ressort à boudin (49) coaxial à l'arbre (38).

Dans les régions encadrant les appendices radiaux (48) du disque (47) on place respectivement un élément photoémetteur (51) et un élément photorécepteur (52) se faisant face et de manière telle que le faisceau lumineux émis par l'élément photoémetteur (51) et dirigé vers l'élément photorécepteur (52) puisse être intercepté par les appendices (48) du disque (47). Les éléments (51) et (52) sont supportés par une petite fourche (53) fixée sur la plaque (8) par un étrier (54). Le signal électrique provenant de l'élément photorécepteur (52), par exemple un phototransistor, parvient à une première entrée d'un bloc d'élaboration (56) dont une seconde entrée reçoit un signal de référence règlable engendré par le bloc (57). Le bloc (56) peut être réalisé de toute manière convenable afin de fournir à une lampe (58) un signal d'alarme chaque fois qu'on atteindra une différence de valeur prédéterminée entre les signaux reçus. En d'autres termes, la lampe (58) fournit à l'opérateur une alarme chaque fois que la rotation de l'arbre (38) qui commande le déplacement du premier élément (17) correspond à des valeurs qui sortent des limites prédéterminées.

En se référant à la figure 2, les moyens de liaison (20) entre les éléments (17) et (19) comprennent essentiellement une vis (61) avec une tête (62), une partie intermédiaire (63) non filetée et une extrémité filetée (64). Celle-ci est vissée dans la partie correspondante (65) d'un trou borgne (66) creusé dans l'élément (19) parallèlement au trou (27) dans la face tournée vers l'élément (17). Le trou borgne (66) reçoit à l'intérieur un ressort à boudin (68) qui est coaxial à la partie intermédiaire (63) de la vis (61). Cette partie intermédiaire peut coulisser axialement à l'intérieur d'un trou (69) creusé dans l'élément (17) à côté du trou (24) précité.

De la même manière la tête (62) de la vis (61) est logée sans interférence à l'intérieur d'une cavité cylindrique (70) coaxiale au trou (69) et tournée du côté opposé à celui qui fait face au ressort (68). Le diamètre de la cavité (70) est plus grand que celui du trou (69) et leurs surfaces cylindriques sont reliées par une paroi annulaire plane et orthogonale (71) qui constitue une butée d'arrêt aux déplacements axiaux de la tête (62) par rapport à l'élément (17) dans le sens de la poussée exercée par le ressort (68).

La longueur de la partie intermédiaire (63) de la vis (61) est essentiellement égale à la somme des longueurs mesurées dans la direction axiale des trous (69), de la partie non filetée du trou borgne (66) et de la course précitée désignée par: a. De cette manière le lien entre le premier élément (17) et le second élément (19) se révèle de type rigide quand, en se référant à la figure 2, l'arbre (38) tourne dans le sens inverse des aiguilles d'une montre afin d'entraîner un déplacement de la droite vers la gauche des éléments (17) et (19). Un tel lien se révèle au contraire de type élastique dans le cas où l'arbre (38) tourne dans le sens des aiguilles d'une montre en fait, dans ces conditions, l'élément (17) transmet un poussée axiale à l'élément (19) au moyen du ressort cylindrique (68). Dans le cas ou l'élément (19) rencontre un obstacle quelconque à la translation axiale de la gauche vers la droite, le ressort à boudin (68) est comprimé par l'élément (17) et la distance entre les éléments (17) et (19) se réduit jusqu'à zero.

Comme on l'a déjà dit, la mesure de la distance entre les éléments (17) et (19) est effectuée par le dispositif optique (22). Le signal électrique émis par celui-ci est envoyé à travers un circuit amplificateur (72) sur la première entrée d'un circuit comparateur (73), dont la seconde entrée est reliée au curseur d'un potentiomètre (74). Celui-ci a une extrémité reliée à la masse et l'autre extrémité reliée à une borne (75) reliée à son tour, d'une manière non représentée, à une source d'alimentation en courant continu. La sortie du comparateur (73) est reliée à des moyens de signalisation (76) comprenant une lampe (77) et un avertisseur acoustique (78).

Le fonctionnement de l'appareil (1) est le suivant. Avant tout le corps (3) de l'injecteur (2) est mis correctement en place grâce à l'élément d'ancrage (14) pendant que le piston (4) est accroché à l'élément (15) en forme de L. On peut ensuite administrer le liquide contenu à l'intérieur de la chambre (5) par le déplacement du piston (4) en alimentant électriquement le motoréducteur (46). Ce dernier entraîne dans le sens des aiguilles d'une montre l'arbre (18), donc dans le même sens l'arbre (38) et par conséquent le déplacement de la gauche vers la droite des éléments (17) et (19), ainsi que de l'élément en L (15) solidaire du piston (4).

La quantité de liquide administrée dépend donc de l'avancement du piston (4) à l'intérieur de la chambre (5) et est contrôlée de la manière indiquée ci-avant par le bloc d'élaboration (56). Ce dernier vérifie en fait que l'avancement des appendices (48) du disque (47) entre les éléments photoémetteur (51) et photorécepteur (52) s'effectue dans des intervalles de temps prédéterminés et imposés à l'avance au moyen du bloc (57) générateur de signaux de référence. Dans le cas où l'avancement des appendices (48) et donc la rotation du disque (47) serait hors des limites de temps prédéterminées, le bloc (56) envoie un signal provoquant l'éclairage de la lampe (58) afin de signaler l'anomalie à l'opérateur.

Dans des conditions normales, l'avancement du piston (4) s'effectue de manière à administrer progressivement le liquide contenu dans la chambre (5) de l'injecteur (2). Dans le cas où l'on observe une obstruction qui empêche ou qui réduit de façon considérable le débit de liquide s'écoulant de la chambre (5), il se crée dans celle-ci une augmentation de pression qui crée une poussée axiale dans la direction opposée au sens d'avancement du piston (4) lui-même. Une telle poussée est transmise à l'élément (15) et de celui-ci, par l'étrier (33) à l'élément (19). Ce dernier en se reportant à la figure 2, tend essentiellement à rester arrêté, provoquant en conséquence la compression du ressort à boudin (68) par l'élément (17). De cette manière les éléments (17) et (19) s'approchent graduellement, ce qui modifie le niveau du signal engendré par le dispositif optique (22). Le signal émis par celui-ci, après être amplifié par le circuit amplificateur (72), est comparé dans le circuit comparateur (73) avec le signal fourni par le potentiomètre (74) et réglé préalablement en vue d'éviter de fausses alarmes.

Lorsque la différence entre les signaux arrivant aux entrées du circuit comparateur (73) devient inférieure à une valeur minimum prédéterminée, le circuit (73) lui-même émet un signal de sortie qui alimente directement la lampe (77) et l'avertisseur acoustique (78) afin d'informer l'opérateur d'une situation anormale. Une situation anormale particulière correspond à la fin de course des éléments (17) et (19) et donc du piston (4); elle est reconnue et signalée lorsque l'arrêt de l'élément (19) est provoqué par la paroi (11) du chassis (7).

A la fin de l'infusion, l'injecteur peut être désaccouplé de l'élément d'ancrage (14) et de l'élément en L (15). Le déplacement de celui-ci peut être enfin commandé par l'opérateur en appliquant une force axiale sur le pommeau (40), pour désaccoupler le disque frontal (42) et en faisant de même tourner l'arbré (38) jusqu'à ce que l'élément (17) rejoigne sa position initiale à proximité de la plaque (10).

De l'examen des caractéristiques de l'appareil construit selon la présente invention, il resulte clairement qu'il permet d'éviter les inconvénients cités ci-avant liés à l'emploi des appareils de type connu. En fait, des obstructions quelconques du conduit d'administration à l'aval de l'injection (2) sont détectées et signalées à l'opérateur alors qu'il est encore possible d'intervenir afin d'éviter tout inconvénient au patient, de même que tout dommage à la ligne d'administration du liquide. Il est important d'observer qu'une telle signalisation n'est pas influencée par l'usure des pièces accouplées entre elles et donc de paramètres qui peuvent varier dans le temps; il en résulte qu'elle est particulièrement sûre et fiable. En outre l'appareil décrit ci-dessus ne nécessite pas l'emploi de micro-interrupteur de signalisation de fin de course du piston, puisque la condition de fin de course est reconnue, comme dit ci-dessus, comme empêchant la poursuite du déplacement du piston et est signalée comme une condition anormale.

Il est clair enfin que l'on peut apporter à l'appareil décrit ci-avant diverses modifications et variantes de réalisation sans pour autant sortir de la présente invention telle que revendiquée. Par exemple les moyens de mesure (22) du déplacement, au lieu d'être de type optico-électronique, pourraient comprendre un transducteur quelconque capable de fournir un signal de sortie électrique dont la valeur dépend de la distance entre les éléments cités (17) et (19). Par ailleurs une solution équivalente à celle décrite ci-avant consisterait à placer le transducteur optique à réflexion sur l'élément (17) plutôt que sur l'élément (19).

**Revendications**

1. Appareil (1) pour administrer un liquide contenu dans la chambre (5) d'un injecteur (2) muni d'un piston (4) coulissant dans ladite chambre (5), l'appareil (1) présentant des moyens d'ancrage du corps (3) de l'injecteur (2) et des moyens de traction du piston (4) en direction axiale à l'intérieur de ladite chambre (5), lesdits moyens de traction comprenant un premier élément (17) accouplé à un organe de transmission de mouvement (18) et un second élément (19) capable de coopérer avec ledit piston (4) pour le tirer axialement, caractérisé en ce que lesdits moyens de traction comprennent:
- des moyens de liaison (20) qui établissent entre lesdits premier et second éléments (17, 19) un accouplement élastique avec une course (a) de long eur prédéterminée dans la direction d'avancement du piston (4) à l'intérieur de ladite chambre (5),
- des moyens (22) permettant de mesurer à tout instant la distance prise suivant la direction d'avancement du piston entre lesdits premier et second éléments, lesdits moyens (22) comprenant un élément transducteur capable d'émettre un signal électrique dépendant de la distance entre lesdits premier et second éléments (17, 19), ledit appareil comprenant en outre des moyens (73) de comparaison dudit signal émis par ledit transducteur (22) avec un signal de référence dont la valeur est fonction d'une distance prédéterminée entre lesdits premier et second éléments (17, 19) pour émettre un signal de sortie, ainsi que des moyens de signalisation (76) reliés à la sortie desdits moyens de comparaison (73) et susceptibles d'être excités ou désexcités par ledit signal de sortie pour indiquer l'obtention de ladite distance prédéterminée.

2. Appareil selon la revendication 1, caractérisé en ce que ledit transducteur (22) est de type optico-électronique.

3. Appareil selon la revendication 2, caractérisé en ce que ledit transducteur de type optico-électronique est constitué essentiellement par un dispositif optique (22) à réflexion fixé sur ledit second élément (19) face audit premier élément (17) ou vice-versa.

4. Appareil selon la revendication 1, caractérisé en ce que lesdits moyens de comparaison comprennent un comparateur à seuil présentant une première entrée recevant ledit signal émis par ledit transducteur (22) et une seconde entrée recevant ledit signal de référence.

5. Appareil selon la revendication 1 ou 4, caractérisé en ce qu'il comprend des moyens de réglage (74) du niveau dudit signal de référence.

6. Appareil selon l'une des revendications précédentes, caractérisé en ce que lesdits moyens de signalisation (76) comprennent un avertisseur optique (77) et/ou un avertisseur acoustique (78).

7. Appareil selon l'une des revendications précédentes, caractérisé en ce qu'il comprend en outre un amplificateur (72) interposé entre ledit transducteur (22) et lesdits moyens de comparaison (73).

8. Appareil selon l'une des revendications précédentes, caractérisé en ce que lesdits moyens de liaison (20) entre lesdits premier (17) et second (18) éléments comprennent essentiellement une vis (61) et un ressort (68), ladite vis (61) présentant une tête (62) capable de coopérer avec une surface correspondante de butée dudit premier élément (17) et une tige avec une partie intermédiaire (63) coulissant par rapport audit premier élément et une extrémité (64) vissée dans le second élément (19) afin de permettre un déplacement axial dudit second élément (19) par rapport audit premier élément (17), ledit ressort étant interposé entre lesdits premier (17) et second (19) éléments.

9. Appareil selon la revendication 8, caractérisé en ce que ledit ressort (68) est du type ressort à boudin et est monté coaxialement à la partie intermédiaire (63) de la tige de ladite vis (61).

10. Appareil selon l'une des revendications précédentes, caractérisé en ce qu'il comprend des moyens (28, 29) de guidage du déplacement axial desdits premier (17) et second (19) éléments.

11. Appareil selon la revendication 10, caractérisé en ce que lesdits moyens de guidage comprennent une paire de guides cylindriques (28, 29) parallèles et fixés à un chassis support (7).

12. Appareil selon la revendication 11, caractérisé en ce que lesdits premier (17) et second (19) éléments ont essentiellement chacun la forme d'un L, et ont chacun une paire de trous parallèles (24, 25; 26, 27) qui coopèrent respectivement avec des guides cylindriques (28, 29).

13. Appareil selon l'une des revendications précédentes, caractérisé en ce que ledit premier élément (17) est solidaire d'une crémaillère (36) qui coopère avec un pignon (37) lequel est accouplé à des moyens de transmission de mouvement (18).

14. Appareil selon la revendication 13, caractérisé en ce qu'il comprend un plateau d'accouplement (42) qui permet l'accouplement en rotation dudit pignon (37) avec lesdits moyens de transmission de mouvement (18).

15. Appareil selon la revendication 14, caractérisé en ce que ledit plateau d'accouplement (42) comprend une seconde partie (45) solidaire desdits moyens de transmission de mouvement (18) et une première partie (44) solidaire dudit pignon (37) et de l'arbre (38) mobile axialement contre l'action de moyens élastiques (49) pour permettre le désaccouplement entre lesdites première (44) et seconde (45) parties du plateau d'accouplement (42).

16. Appareil selon la revendication 15, caractérisé en ce que lesdits moyens élastiques (49) comprennent un ressort à boudin (49) coaxial à l'arbre (38) comprimé entre un élément fixe (8) solidaire du chassis dudit appareil et un élément (47) solidaire dudit arbre (38).

17. Appareil selon la revendication 15 ou 16, caractérisé en ce que ledit arbre (38) s'étend à l'extérieur du chassis dudit appareil pour être engagé dans un pommeau (40) manoeuvrable à la main pour entraîner en translation axiale et en rotation ledit arbre (38).

18. Appareil selon l'une des revendications précédentes, caractérisé en ce qu'il comprend des moyens pour relever et contrôler le déplacement dudit premier élément (17) sous l'action des moyens de transmission de mouvement (18).

19. Appareil selon la revendication 18, caractérisé en ce que lesdits moyens pour relever et contrôler ledit déplacement comprennent essentiellement des moyens optico-électroniques (51, 52) capables d'émettre un signal électrique en fonction de l'avancement dudit premier élément (17), et des moyens d'élaboration (56) capables de comparer ledit signal électrique avec un signal correspondant de référence et à émettre un signal d'alarme quand l'écartement entre le signal dépendant de la position du premier élément (17) et le signal de référence sort des limites prédéterminées.

20. Appareil selon la revendication 19, caractérisé en ce que lesdits moyens de mesure (51, 52) coopèrent avec les appendices radiaux (48) d'un disque (47) solidaire dudit arbre (38) portant ledit pignon (37).

**Patentansprüche**

1. Gerät (1) zum Verabreichen einer Flüssigkeit, die in der Kammer (5) einer Spritze (2) enthalten ist, die einen Kolben (4) aufweist, der verschiebbar in der Kammer (5) angeordnet ist, wobei das Gerät eine Vorrichtung zum Befestigen des Körpers (3) der Spritze (2) und eine Vorrichtung zum Verschieben des Kolbens (4) in Axialrichtung ins Innere der Kammer (5) aufweist, wobei die Vorrichtung zum Verschieben ein erstes Element (17), das mit einem Element (18) zur Übertragung der Verschiebung gekoppelt ist, und ein zweites Element (19) aufweist, welches mit dem Kolben (4) zusammenwirken kann, um diesen in Axialrichtung zu ziehen, dadurch gekennzeichnet, daß die Vorrichtung zum Verschieben aufweist:

- eine Verbindungseinrichtung (20), welche zwischen dem ersten und dem zweiten Element (17, 19) eine elastische Kupplung mit einem Hub (a) einer bestimmten Länge in Richtung des Vorschubs des Kolbens (4) ins Innere der Kammer (5) erbringt,

- eine Einrichtung (22), mit welcher jederzeit der eingenommene Abstand gemäß der Vorschubrichtung des Kolbens zwischen dem ersten und dem zweiten Element gemessen werden kann, wobei die Einrichtung (22) ein Meßwandlerelement umfaßt, das ein elektrisches Signal abhängig von dem Abstand zwischen dem ersten und dem zweiten Element (17, 19) liefern kann,

wobei das Gerät ferner eine Einrichtung (73) zum Vergleichen des Signals, das von dem Meßwandler (22) geliefert wird, mit einem Referenzsignal, dessen Wert eine Funktion eines vorbestimmten Abstandes zwischen dem ersten und dem zweiten Element (17, 19) ist, um ein Ausgangssignal zu liefern, sowie eine Signaleinrichtung (76) aufweist, die mit dem Ausgang der Vergleichseinrichtung (73) verbunden ist und die von dem Ausgangssignal erregt oder aberregt werden kann, um die Erreichung des vorbestimmten Abstandes aufzuzeigen.

2. Gerät nach Anspruch 1, dadurch gekennzeichnet, daß der Meßwandler (22) vom optischelektronischen Typ ist.

3. Gerät nach Anspruch 2, dadurch gekennzeichnet, daß der Meßwandler vom optischelektronischen Typ im wesentlichen von einer optischen Reflektionseinrichtung (22) gebildet wird, die auf dem zweiten Element (19) gegenüber dem ersten Element (17) angeordnet ist oder umgekehrt.

4. Gerät nach Anspruch 1, dadurch gekennzeichnet, daß die Vergleichseinrichtung einen Schwellenvergleicher aufweist mit einem ersten Eingang, der das von dem Meßwandler (22) gelieferte Signal aufnimmt, und mit einem zweiten Eingang, der das Referenzsignal aufnimmt.

5. Gerät nach Anspruch 1 oder 4, dadurch gekennzeichnet, daß es eine Vorrichtung (74) zum Regeln der Höhe des Referenzsignals aufweist.

6. Gerät nach einem der vorhergehenden Ansprüche, dadurch gekennzeichnet, daß die Signaleinrichtung (76) eine optische Alarmvorrichtung (77) und/oder eine akkustische Alarmvorrichtung (78) aufweist.

7. Gerät nach einem der vorhergehenden Ansprüche, dadurch gekennzeichnet, daß es ferner einen Verstärker (72) aufweist, der zwischen dem Meßwandler (22) und der Vergleichseinrichtung (73) angeordnet ist.

8. Gerät nach einem der vorhergehenden Ansprüche, dadurch gekennzeichnet, daß die Verbindungseinrichtung (20) zwischen dem ersten (17) und dem zweiten (18) Element im wesentlichen eine Schraube (61) und eine Feder (68) aufweist, daß die Schraube (61) einen Kopf (62), der mit einer entsprechenden Anschlagfläche an dem ersten Element (17) zusammenwirkt, einen Schaft mit einem Zwischenstück (63), das gegenüber dem ersten Element verschiebbar ist, und ein Ende (64) aufweist, das in dem zweiten Element (19) eingeschraubt ist, um eine Axialverschiebung des zweiten Elementes (19) gegenüber dem ersten Element (17) zu ermöglichen, und daß die Feder zwischen dem ersten (17) und dem zweiten (19) Element angeordnet ist.

9. Gerät nach Anspruch 8, dadurch gekennzeichnet, daß die Feder (68) vom Typ einer Schraubenfeder und koaxial zu dem Zwischenstück (63) des Schaftes der Schraube (61) angeordnet ist.

10. Gerät nach einem der vorhergehenden Ansprüche, dadurch gekennzeichnet, daß eine Vorrichtung (28, 29) zur Führung der Axialverschiebung des ersten (17) und des zweiten (19) Elementes vorgesehen ist.

11. Gerät nach Anspruch 10, dadurch gekennzeichnet, daß die Führungsvorrichtung ein Paar zylindrischer, paralleler und an einem Tragrahmen (7) befestigter Führungen (28, 29) aufweist

12. Gerät nach Anspruch 11, dadurch gekennzeichnet, daß das erste (17) und das zweite (19) Element jeweils im wesentlichen die Form eines L aufweisen und daß jedes der Elemente ein Paar paralleler Löcher (24, 25; 26, 27) aufweist, welche jeweils mit den zylindrischen Führungen (28, 29) zusammenwirken.

13. Gerät nach einem der vorhergehenden Ansprüche, dadurch gekennzeichnet, daß das erste Element (17) fest mit einer Zahnstange (36) verbunden ist, die mit einem Ritzel (37) zusammenwirkt, welches an der Vorrichtung (18) zur Übertragung der Bewegung gekoppelt ist.

14. Gerät nach Anspruch 13, dadurch gekennzeichnet, daß eine Kopplungsplatte (42) vorgesehen ist, welche die Kopplung zum Drehen des Ritzels (37) mit der Einrichtung (18) zur Übertragung der Bewegung ermöglicht.

15. Gerät nach Anspruch 14, dadurch gekennzeichnet, daß die Kopplungsplatte (42) ein erstes Teil (45), welches fest mit der Einrichtung (18) zur Übertragung der Bewegung verbunden ist, und ein zweites Teil (44), das fest mit dem Ritzel (37) verbunden ist, umfaßt, und daß eine Welle (38) vorgesehen ist, die in Axialrichtung beweglich ist entgegen der Kraft einer elastischen Einrichtung (49), um das Abkoppeln zwischen dem ersten (44) und dem zweiten (45) Teil der Kopplungsplatte (42) zu ermöglichen.

16. Gerät nach Anspruch 15, dadurch gekennzeichnet, daß die elastische Einrichtung (49) eine Schraubenfeder (49) umfaßt, die koaxial zu der Welle (38) angeordnet ist und die zwischen einem festen Element (8), das an dem Rahmen des Gerätes befestigt ist, und einem Element (47), das fest an der Welle (38) ist, zusammengedrückt wird.

17. Gerät nach Anspruch 15 oder 16, dadurch gekennzeichnet, daß die Welle (38) sich nach außen von dem Rahmen des Gerätes erstreckt, um in einen Knopf (40) einzugreifen, der von Hand betätigbar ist, um eine Axialverschiebung und eine Drehung der Welle (38) durchzuführen.

18. Gerät nach einem der vorhergehenden Ansprüche, dadurch gekennzeichnet, daß eine Einrichtung zum Aufnehmen und Steuern der Verschiebung des ersten Elementes (17) unter der Betätigung der Einrichtung (18) zur Übertragung der Bewegung vorgesehen ist.

19. Gerät nach Anspruch 18, dadurch gekennzeichnet, daß die Einrichtung zum Aufnehmen und Steuern der Verschiebung im wesentlichen eine optisch-elektronische Einrichtung (51, 52), die ein elektrisches Signal als Funktion des Vorschubs des ersten Elementes (17) aussenden kann, und eine Verarbeitungseinrichtung (56) umfaßt, um das elektrische Signal mit einem entsprechenden Referenzsignal zu vergleichen und ein Alarmsignal zu liefern, wenn der Abstand zwischen dem Signal, das abhängig ist von der Stellung des ersten Elementes (17), und dem Referenzsignal vorbestimmte Grenzen verläßt.

20. Gerät nach Anspruch 19, dadurch gekennzeichnet, daß die Einrichtung (51, 52) zum Messen mit radialen Ansatzstücken (48) einer Scheibe (47) zusammenwirkt, die fest an der Welle (38) angeordnet ist, an welcher das Ritzel (37) vorgesehen ist.

**Claims**

1. Apparatus (1) for administering a liquid contained in the chamber (5) of an injector (2) equipped with a piston (4) sliding in the said chamber (5), the apparatus (1) exhibiting means for anchoring the body (3) of the injector (2) and means for the traction of the piston (4) in the axial direction within the said chamber (5), the said traction means comprising a first element (17) coupled to a component for the transmission of movement (18) and a second element (19) capable of cooperating with the said piston (4) to draw it axially, characterized in that the said traction means comprise:

- linkage means (20) which establish between the said first and elements (17, 19) a flexible coupling with a stroke (a) of predetermined length in the direction of advance of the piston (4) within the said chamber (5),

- means (22) permitting the measurement, at any instant, of the distance taken along the direction of advance of the piston between the said first and second elements, the said means (22) comprising a transducer element capable of emitting an electrical signal dependent upon the distance between the said first and second elements (17, 19), the said apparatus further comprising means (73) for comparison of the said signal emitted by the said transducer (22) with a reference signal, the value of which is a function of a predetermined distance between the said first and second elements (17, 19) to emit an output signal, as well as signalling means (76) connected to the output of the said comparison means (73) and capable of being energized or deenergized by the said output signal to indicate that the said predetermined distance has been reached.

2. Apparatus according to claim 1, characterized in that the said transducer (22) is of the optoelectronic type.

3. Apparatus according to claim 2, characterized in that the said transducer of the optoelectronic type consists essentially of an optical reflection device (22) fixed on the said second element (19) facing the said first element (17) or vice versa.

4. Apparatus according to claim 1, characterized in that the said comparison means comprise a threshold comparator exhibiting a first input receiving the said signal emitted by the said transducer (22) and a second input receiving the said reference signal.

5. Apparatus according to claim 1 or 4, characterized in that it comprises means (74) for regulating the level of the said reference signal.

6. Apparatus according to one of the preceding claims, characterized in that the said signalling means (76) comprise an optical warning device (77) and/or an acoustic warning device (78).

7. Apparatus according to one of the preceding claims, characterized in that it further comprises an amplifier (72) interposed between the said transducer (22) and the said comparison means (73).

8. Apparatus according to one of the preceding claims, characterized in that the said linkage means (20) between the said first (17) and second (18) elements essentially comprise a screw (61) and a spring (68), the said screw (61) exhibiting a head (62) capable of cooperating with a corresponding abutment surface of the said first element (17) and a rod with an intermediate part (63) sliding in relation to the said first element and an end (64) screwed into the second element (19) in order to permit an axial displacement of the said second element (19) in relation to the said first element (17), the said spring being interposed between the said first (17) and second (19) elements.

9. Apparatus according to claim 8, characterized in that the said spring (68) is of the coil-spring type and is mounted coaxially with the intermediate part (63) of the rod of the said screw (61).

10. Apparatus according to one of the preceding claims, characterized in that it comprises means (28, 29) for guiding the axial displacement of the said first (17) and second (19) elements.

11. Apparatus according to claim 10, characterized in that the said guiding means comprise a pair of cylindrical guides (28, 29) which are parallel and fixed to a supporting frame (7).

12. Apparatus according to claim 11, characterized in that the said first (17) and second (19) elements each have essentially the shape of an L, and each have a pair of parallel holes (24, 25; 26, 27) which cooperate respectively with cylindrical guides (28, 29).

13. Apparatus according to one of the preceding claims, characterized in that the said first element (17) is integral with a rack (36) which cooperates with a pinion (37) which is coupled to means for transmission of movement (18).

14. Apparatus according to claim 13, characterized in that it comprises a coupling plate (42) which permits the coupling, in rotation, of the said pinion (37) with the said means for transmission of movement (18).

15. Apparatus according to claim 14, characterized in that the said coupling plate (42) comprises a second part (45) integral with the said means for transmission of movement (18) and a first part (44) integral with the said pinion (37) and with the shaft (38) movable axially against the action of resilient means (49) to permit the decoupling between the said first (44) and second (45) parts of the coupling plate (42).

16. Apparatus according to claim 15, characterized in that the said resilient means (49) comprise a coil spring (49) coaxial with the shaft (38) and compressed between a fixed element (8) integral with the frame of the said apparatus and an element (47) integral with the said shaft (38).

17. Apparatus according to claim 15 or 16, characterized in that the said shaft (38) extends outside the frame of the said apparatus to be engaged in a cover (40) which is manually operable to drive the said shaft (38) in axial translation and in rotation.

18. Apparatus according to one of the preceding claims, characterized in that it comprises means for determining and monitoring the displacement of the said first element (17) under the action of the means for transmission of movement (18).

19. Apparatus according to claim 18, characterized in that the said means for determining and monitoring the said displacement essentially comprise optoelectronic means (51, 52) capable of emitting an electrical signal as a function of the advance of the said first element (17), and processing means (56) capable of comparing the said electrical signal with a corresponding reference signal and of emitting an alarm signal when the difference between the signal dependent upon the position of the first element (17) and the reference signal departs from the predetermined limits.

20. Apparatus according to claim 19, characterized in that the said measuring means (51, 52) cooperate with the radial appendices (48) of a disc (47) integral with the said shaft (38) carrying the said pinion (37).

Fig. 1.

Fig.2.

EP 0 171 337 B1